(19) Europäisches Patentamt
European Patent Office
Office européen des brevets

(11) Numéro de publication: 0 046 417
B1

# (12) FASCICULE DE BREVET EUROPEEN

(45) Date de publication du fascicule du brevet: **11.01.84**

(51) Int. Cl.³: **C 07 D 409/04**, C 07 D 411/04, C 07 D 405/04, A 61 K 31/395

(21) Numéro de dépôt: **81400381.0**

(22) Date de dépôt: **12.03.81**

(54) **Nouveaux dérivés de la thioformamide, leur préparation et les médicaments qui les contiennent.**

(30) Priorité: **18.08.80 FR 8018035**

(43) Date de publication de la demande: **24.02.82 Bulletin 82/8**

(45) Mention de la délivrance du brevet: **11.01.84 Bulletin 84/2**

(84) Etats contractants désignés: **AT BE CH DE FR GB IT LI LU NL SE**

(56) Documents cités:
**FR - A - 2 100 970**
**FR - A - 2 258 178**
**FR - A - 2 452 488**

**Le dossier contient des informations techniques présentées postérieurement au dépôt de la demande et ne figurant pas dans le présent fascicule.**

(73) Titulaire: **RHONE-POULENC SANTE, Les Miroirs 18 Avenue d'Alsace, F-92400 Courbevoie Cedex (FR)**

(72) Inventeur: **Aloup, Jean-Claude, 53, rue Marcel Risser, F-94290 Villeneuve-Le-Roi (FR)**
Inventeur: **Bouchaudon, Jean, 23, Avenue des Saules, 91390 Morsang-Sur-Orge (FR)**
Inventeur: **Farge, Daniel, 30, Rue des Pins Sylvestres, F-94320 Thiais (FR)**
Inventeur: **James, Claude, 31, bis avenue Gambetta, 75020 Paris (FR)**

(74) Mandataire: **Gaumont, Robert et al, RHONE-POULENC RECHERCHES Service Brevets Pharma 25, Quai Paul Doumer, F-92408 Courbevoie Cedex (FR)**

EP 0 046 417 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Nouveaux dérivés de la thioformamide, leur préparation et les médicaments qui les contiennent.

La présente invention concerne de nouveaux dérivés de la thioformamide de formule générale:

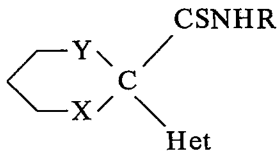

(I)

Dans la formule générale (I), R représente un atome d'hydrogène ou un radical alcoyle contenant 1 à 4 atomes de carbone en chaîne droite ou ramifiée et

- soit Het représente le radical quinolyle-2, X représente un atome de soufre et Y représente une liaison de valence,
- soit Het représente le radical pyridyle-2, X représente un atome de soufre ou d'oxygène et Y représente un atome de soufre ou d'oxygène ou un radical méthylène,
- soit Het représente le radical pyridyle-2, X représente un atome d'oxygène et Y représente un liaison de valence.

On connaît déjà par les demandes de brevets français publiées sous les numéros 2 100 970 et 2 258 178 des dérivés de la thioacétamide et d'acides hétérocyclylalcanethiocarboxyliques qui inhibent la sécrétion gastrique. Aucun de ces documents ne décrit les produits de formule générale (I) dans laquelle X et Y ont les définitions ci-dessus, ni ne suggère que ces produits ont une activité antisécrétoire supérieure. On connaît également par le brevet français publiés sous le numéro 2 452 488 des (pyridyl-2)-2 tétrahydrothiophènecarbothioamide-2 utiles comme anti-ulcères et antisécrétoires; ce brevet, non accessible au public à la date de priorité de la présente demande, décrit des produits différents de ceux de la formule générale (I) ci-dessus.

Selon l'invention, les produits de formule générale (I) dans laquelle les symboles R, Het, X et Y sont définis comme précédemment peuvent être préparés par action d'une amine de formule générale:

$$R-NH_2 \qquad (II)$$

dans laquelle R est défini comme précedemment sur un dithioester de formule générale:

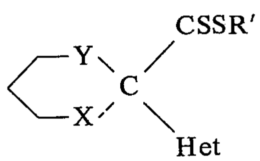

(III)

dans laquelle les symboles Het, X et Y sont définis comme précédemment et R' représente un radical alcoyle contenant 1 à 4 atomes de carbone en chaîne droite ou ramifiée ou un radical benzyle ou carboxyméthyle.

Généralement, on opère avec un excès d'amine de formule générale (II), sans solvant ou dans un solvant organique tel qu'un carbure aromatique, un éther ou un alcool à bas poids moléculaire ou un mélange de ces solvants, à une température comprise entre 20 et 130°C, éventuellement sous pression.

Les dithioesters de formule générale (III) peuvent être obtenus par action d'un dérivé organo-lithien sur un produit de formule générale:

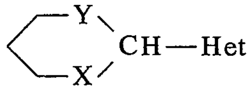

(IV)

dans laquelle Het, X et Y sont définis comme précédemment, suivie de l'action du sulfure de carbone puis d'un produit de formule générale:

$$R'-Z \qquad (V)$$

dans laquelle R' est défini comme précédemment et Z représente un atome d'halogène, de préférence un atome de chlore, de brome, ou d'iode ou un reste d'ester réactif, de préférence un reste mésyloxy ou tosyloxy.

La réaction s'effectue généralement dans un solvant organique anhydre tel que l'hexaméthylphos-

phorotriamide, additionné généralement d'un éther tel que le tétrahydrofuranne, à une température comprise entre −80 et −40°C.

Les dérivés organo-lithiens qui conviennent particulièrement bien sont de préférence les alcoylures de lithium tels que le butyllithium et l'isopropyllithium ou le phényllithium, en solution dans un solvant inerte comme l'hexane, ou les amidures de lithium tels que le diéthylamidure de lithium ou le diisopropylamidure de lithium.

Selon les définitions de X et de Y, les produits de formule générale (IV) peuvent être préparés comme suit:

a) Les produits de formule générale (IV) dans laquelle

— ou bien Het représente le radical quinolyle-2, X représente un atome de soufre et Y représente une liaison de valence, c'est-à-dire le produit de formule:

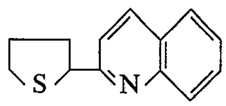

(VI)

— ou bien Het représente le radical pyridyle-2, X représente un atome de soufre et Y représente un radical méthylène, c'est-à-dire le produit de formule:

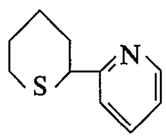

(VII)

peuvent être préparés par cyclisation au moyen d'une base organique, telle qu'un alcoolate ou un amidure alcalin, d'un dérivé de formule générale:

$$Het_2-CH_2S(CH_2)_3-Y-Z° \qquad (VIII)$$

dans laquelle $Het_2$ représente le radical quinolyle-2 ou pyridyle-2, X et Y sont définis comme il vient d'être dit et Z° représente un atome d'halogène de préférence de chlore ou de brome ou un reste d'ester réactif, de préférence un reste mésyloxy ou tosyloxy, en opérant au sein d'un solvant organique anhydre, tel que le tétrahydrofuranne ou l'hexaméthylphosphorotriamide ou un mélange de ces solvants, à une température comprise entre −80 et +25°C. Comme base organique, il est particulièrement avantageux d'utiliser le t.butylate de potassium, le diéthylamidure de lithium ou le diisopropylamidure de lithium.

Les dérivés hétérocycliques de formule (VIII) peuvent être obtenus par hydrolyse alcaline, de préférence au moyen d'une solution aqueuse d'un hydroxyde alcalin tel que la soude, d'un sel d'une isothiourée de formule générale:

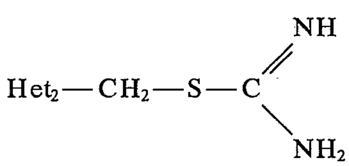

(IX)

à une température comprise entre 50°C et la température d'ébullition du mélange réactionnel, suivie de l'action d'un produit de formule générale:

$$Z'-(CH_2)_3-Y-Z' \qquad (X)$$

dans laquelle Y est défini comme précédemment et les symboles Z', identiques ou différents, représentent chacun un atome d'halogène de préférence atome de chlore ou de brome, ou un reste d'ester réactif, de préférence un reste mésyloxy ou tosyloxy, à une température voisine de 20°C en présence d'un hydroxyde alcalin tel que la soude.

Il est possible d'isoler intermédiairement le dérivé hétérocyclique de formule générale:

$$Het_2-CH_2-SH \qquad (XI)$$

provenant de l'hydrolyse alcaline de l'isothiourée de formule générale (IX), puis de faire réagir le produit de formule générale (X) en présence d'un hydroxyde alcalin tel que la soude.

Les isothiourées de formule générale (XI), sous forme de sels tels que chlorhydrates, peuvent être obtenues par action de la thiourée sur un dérivé hétérocyclique de formule générale:

$$Het_2-CH_2-Z'' \qquad (XII)$$

dans laquelle Z'' représente un atome d'halogène, de préférence un atome de chlore ou de brome, éventuellement sous forme d'un sel tel qu'un halohydrate, en opérant dans un solvant organique tel qu'un alcool (éthanol) à la température de reflux du mélange réactionnel.

Les dérivés hétérocycliques de formule (XII) peuvent être préparés en employant, selon l'hétérocycle choisi, soit la méthode de W. MATHES et H. SCHÜLY, Angew. Chem. Intern. Ed. 2, 144 (1963), soit la méthode de H. S. MOSHER et J. E. TESSIERI, J. Am. Chem. Soc. 73, 4925 (1957), soit encore celles de K. Y. NOVOTSKII et coll., Khim. Getérotsikl. Soedin. (3), 412 (1970); C. A. 73, 25385z (1970) ou A. HIRSCHBERG et P. E. SPOERRI, J. Org. Chem. 26, 2356 (1961).

b) Les produits de formule générale (IV) dans laquelle Het est défini comme précédemment, X représente un atome de soufre ou d'oxygène et Y représente un atome de soufre ou d'oxygène peuvent être préparés par action d'un dérivé de formule générale:

$$HX-(CH_2)_3-Y-H \qquad (XIII)$$

sur un aldéhyde de formule générale:

$$Het-CHO \qquad (XIV)$$

dans un solvant permettant d'éliminer par distillation azéotropique l'eau formée en cours de réaction. Dans la pratique, on utilise de préférence le benzène, le toluène, les xylènes ou le dichloro-1,2 éthane.

c) Les produits de formule générale (IV) dans laquelle Het est défini comme précédemment, X représente un atome d'oxygène et Y représente une liaison de valence ou un radical méthylène peuvent être préparés par cyclisation d'un produit de formule générale:

$$Het-CHOH(CH_2)_nO-R'' \qquad (XV)$$

dans laquelle R'' est un radical protecteur de fonction alcool tel qu'un radical t.butyle, t.pentyle ou tétrahydropyrannyle et n est égal à 3 ou 4. On opère généralement par chauffage à reflux dans un solvant tel que le toluène en présence d'acide paratoluènesulfonique; ce traitement peut être éventuellement suivi d'un chauffage dans l'acide polyphosphorique à une température comprise entre 50 et 120° C.

Les produits de formule générale (XV) peuvent être préparés par action d'un dérivé magnésien de formule générale:

$$Z'''Mg-(CH_2)_nO-R'' \qquad (XVI)$$

dans laquelle R'' et n sont définis comme ci-dessus et Z''' représente un atome d'halogène tel qu'un atome de brome ou d'iode, sur un aldéhyde de formule générale (XV), en opérant par analogie avec la méthode décrite par W. B. RENFROW, J. Org. Chem. 26, 935 (1961).

Selon l'invention, les produits de formule générale (I) dans laquelle Het, X et Y sont définis comme précédemment et R représente un radical alcoyle contenant 1 à 4 atomes de carbone en chaîne droite ou ramifiée peuvent être obtenus par action d'un dérivé organo-lithien sur un produit de formule générale (IV) suivie de l'action d'un isothiocyanate de formule générale:

$$R'''-N=C=S \qquad (XVII)$$

dans laquelle R''' représente un radical alcoyle contenant 1 à 4 atomes de carbone en chaîne droite ou ramifiée.

Les dérivés organolithiens qui conviennent particulièrement bien sont de préférence les alcoylures de lithium tels que le butyllithium et l'isopropyllithium ou le phényllithium, en solution dans un solvant inerte comme l'hexane, ou les amidures tels que le diéthylamidure de lithium ou le diisopropylamidure de lithium.

Lorsque le produit de formule générale (IV) est obtenu, comme il a été dit précédemment en a), à partir d'un produit de formule générale (VIII) et d'un amidure alcalin, il n'est pas nécessaire d'isoler le produit de formule générale (IV) avant de faire réagir l'isothiocyanate. Il suffit d'employer deux équivalents d'amidure.

La réaction s'effectue généralement dans un solvant organique anhydre tel que l'hexaméthylphos-

phorotriamide généralement additionné d'un éther tel que le tétrahydrofuranne, à une température comprise entre −80 et −40° C.

Les nouveaux produits selon la présente invention peuvent être purifiés par les méthodes physiques habituelles, notamment la cristallisation et la chromatographie.

Les nouveaux produits selon l'invention présentent des propriétés pharmacologiques particulièrement intéressantes associés à une faible toxicité. Ils manifestent une activité anti-ulcère et une activité anti-sécrétoire. Ces propriétés peuvent être mises en évidence chez le rat, à des doses comprises entre 1 et 100 mg/kg par voie orale, en particulier dans la technique de ROSSI et coll. C. R. Soc. Biol., 150, 2124 (1956) et celle de SHAY et coll., Gastroenterology, 5, 43 (1945). Leur dose letale ($DL_{50}$) chez la souris, est généralement supérieure à 300 mg/kg par voie orale.

Sont plus spécialement intéressants les produits de formule générale (I) dans laquelle le symbole Het tel qu'il vient d'être défini, représente le radical pyridyle-2 ou quinolyle-2.

Sont d'un intérêt tout particulier:

— le N-méthyl (quinolyl-2)-2 tétrahydrothiophènecarbothioamide-2
— le N-méthyl (pyridyl-2)-2 tétrahydrothiopyrannecarbothioamide-2
— le N-méthyl (pyridyl-2)-2 dithianne-1,3 carbothioamide-2
— le N-méthyl (pyridyl-2)-2 tétrahydrofurannecarbothioamide-2
— le (pyridyl-2)-2 dithianne-1,3 carbothioamide-2

Les exemples suivants, donnés à titre non limitatif, montrent comment l'invention peut être mise en pratique.

Dans les exemples qui suivent, les chromatographies ont été effectuées avec de la silice ayant pour granulométrie 0,063—0,20 mm ou de l'alumine ayant pour granulométrie 0,125—0,15 mm.

## Exemple 1

A une suspension de 20 g de (quinolyl-2)-2 tétrahydrothiophènecarbodithioate-2 de méthyle dans 42 $cm^3$ d'éthanol maintenue à une température voisine de 20° C, on ajoute goutte à goutte et en 10 minutes 13,5 $cm^3$ d'une solution à 33% (poids/volume) de méthylamine dans l'éthanol. Le mélange réactionnel est ensuite agité pendant 15 heures à une température voisine de 20° C. Les cristaux apparus sont séparés par filtration, lavés 2 fois par 24 $cm^3$ au total d'éthanol et séchés sous pression réduite (20 mm de mercure; 2,7 kPa) à une température voisine de 20° C. On obtient ainsi 12,1 g de produit brut cristallisé. On redissout ce produit dans 75 $cm^3$ d'éthanol bouillànt; la solution est additionnée de 0,6 g de noir décolorant, filtrée à chaud puis refroidie pendant 3 heures à une température voisine de 5° C. Les cristaux apparus sont séparés par filtration, lavés 2 fois par 24 $cm^3$ au total d'éthanol et séchés sous pression réduite (1 mm de mercure; 0,13 kPa) à 60° C. On obtient ainsi 11 g de N-méthyl (quinolyl-2)-2 tétrahydrothiophènecarbothioamide-2 fondant à 124° C.

Le (quinolyl-2)-2 tétrahydrothiophènecarbodithioate-2 de méthyle peut être préparé de la manière suivante:

A 138 $cm^3$ d'une solution 1,6 M de n.butyllithium dans l'hexane, maintenue sous atmosphère d'argon à une température voisine de −60° C, on ajoute goutte à goutte et en 10 minutes 100 $cm^3$ d'un mélange d'hexaméthylphosphorotriamide et de tétrahydrofuranne anhydres (47—53 en volumes). On ajoute ensuite en 15 minutes une solution de 31 g de (quinolyl-2)-2 tétrahydrothiophène dans 100 $cm^3$ du mélange d'hexaméthylphosphorotriamide et de tétrahydrofuranne anhydres (47—53 en volumes). Après 5 minutes d'agitation, on ajoute goutte à goutte, en 15 minutes et à la même température, une solution de 17 g de sulfure de carbone dans 100 $cm^3$ du mélange d'hexaméthylphosphorotriamide et de tétrahydrofurane anhydres (47—53 en volumes). Après 5 minutes d'agitation, on ajoute goutte à goutte et en 25 minutes, une solution de 32 g d'iodure de méthyle dans 100 $cm^3$ du mélange d'hexaméthylphosphorotriamide et de tétrahydrofuranne anhydres (47—53 en volumes). Le mélange réactionnel est ensuite agité pendant 45 minutes à −65° C puis pendant 45 minutes en laissant remonter progressivement la température à 20° C environ. Il est ensuite coulé dans un mélange de 750 $cm^3$ d'eau distillée et de 450 $cm^3$ d'acétate d'éthyle. Après décantation, la phase aqueuse est extraite par 250 $cm^3$ d'acétate d'éthyle. Les extraits organiques sont réunis, lavés 3 fois par 2250 $cm^3$ au total d'eau distillée, séchés sur du sulfate de sodium anhydre, filtrés et concentrés à sec sous pression réduite (20 mm de mercure; 2,7 kPa) à 70° C. Le produit obtenu (57 g) est chromatographié sur 550 g de gel de silice neutre contenus dans une colonne de 5,2 cm de diamètre; la colonne est ensuite éluée par 1000 $cm^3$ d'un mélange cyclohexane-acétate d'éthyle (95—5 en volumes) et par 1000 $cm^3$ d'un mélange cyclohexaneacétate d'éthyle (90—10 en volumes) en recueillant des fractions de 500 $cm^3$. Les fractions 1 à 3 sont réunies et concentrées à sec sous pression réduite (20 mm de mercure; 2,7 kPa) à 50° C; on obtient ainsi 36,6 g de produit brut. On en dissout 14 g dans 70 $cm^3$ d'oxyde d'isopropyle bouillant; la solution est additionnée de 0,3 g de noir décolorant, filtrée à chaud puis refroidie pendant 2 heures à une température voisine de 5° C. Les cristaux apparus sont séparés par filtration, lavés par 7 $cm^3$ d'oxyde d'isopropyle puis 2 fois par 30 $cm^3$ au total d'éther de pétrole. Après séchage sous pression

réduite (1 mm de mercure; 0,13 kPa) à 45° C, on obtient 8,9 g de (quinolyl-2)-2 tétrahydrothiophènecarbodithioate-2 de méthyle fondant à 72° C.

Le (quinolyl-2)-2 tétrahydrothiophène peut être préparé de la manière suivante:

Une solution de 82 g de sulfure de quinolyl-2 méthyle et de chloro-3 propyle dans 100 $cm^3$ de tétrahydrofuranne anhydre est ajoutée goutte à goutte en 35 minutes en maintenant la température en-dessous de 33° C à une solution de 56,5 g de tertiobutylate de potassium dans un mélange de 85 $cm^3$ d'hexaméthylphosphorotriamide et de 455 $cm^3$ de tétrahydrofuranne anhydres. Le mélange réactionnel est ensuite agité pendant 1 heure 15 minutes à la même température puis est coulé sur un mélange de 800 $cm^3$ d'eau distillée et de 500 $cm^3$ d'éther éthylique. Après décantation, la phase aqueuse est extraite 2 fois par 320 $cm^3$ d'éther éthylique. Les extraits éthérés sont réunis, lavés 3 fois par 2400 $cm^3$ au total d'eau distillée, séchés sur du sulfate de sodium anhydre, filtrés et concentrés sous pression réduite (20 mm de mercure; 2,7 kPa) à 35°C. Le produit obtenu (50 g) est chromatographié sur 500 g de gel de silice neutre contenus dans une colonne de 5 cm de diamètre; la colonne est éluée par 2000 $cm^3$ d'un mélange cyclohexane-acétate d'éthyle (95—5 en volumes) puis par 1000 $cm^3$ d'un mélange cyclohexane-acétate d'éthyle (90-10 en volumes) en recueillant des fractions de 500 $cm^3$. Les fractions 3 et 4 sont réunies et concentrées à sec sous pression réduite (20 mm de mercure; 2,7 kPa) à 40° C; on obtient ainsi 31 g de produit cristallisé utilisé tel quel dans la réaction décrite précédemment. Pour identification à l'état pur, on redissout 0,3 g de ce produit dans 1,5 $cm^3$ d'oxyde d'isopropyle bouillant et refroidit la solution obtenue pendant 30 minutes à une température voisine de 0° C. Les cristaux apparus sont séparés par filtration, lavés par 0,5 $cm^3$ d'oxyde d'isopropyle et séchés sous pression réduite (20 mm de mercure; 2,7 kPa) à 20° C. On obtient ainsi 0,2 g de (quinolyl-2)-2 tétrahydrothiophène fondant à 63° C.

Le sulfure de quinolyl-2 méthyle et de chloro-3 propyle peut être préparé de la manière suivante:

A une solution de 141 g de dichlorhydrate de (quinolyl-2 méthyl)-2 isothiourée dans 240 $cm^3$ d'eau distillée, on ajoute goutte à goutte à 15°C et en 15 minutes 97 $cm^3$ d'une solution aqueuse de soude 10 N. Après chauffage pendant 20 minutes à 73° C puis refroidissement à 10° C, on ajoute sous agitation 60 $cm^3$ d'une solution aqueuse de soude 10 N puis 81,5 g de bromo-1 chloro-3 propane et poursuit l'agitation pendant 15 heures à une température voisine de 20° C. Le mélange réactionnel est alors extrait 3 fois par 430 $cm^3$ au total de chlorure de méthylène; les extraits organiques sont réunis, lavés par 250 $cm^3$ d'eau distillée et séchés sur du sulfate de sodium anhydre. Après filtration, la solution obtenue est versée sur 250 g de gel de silice neutre contenus dans une colonne de 4,3 cm de diamètre; la colonne est ensuite éluée par 1180 $cm^3$ de chlorure de méthylène. La première fraction (700 $cm^3$) est éliminée. La seconde fraction (480 $cm^3$) est recueillie et concentrée à sec sous pression réduite (20 mm de mercure; 2,7 kPa) à 35° C. On obtient ainsi 82 g de sulfure de quinolyl-2 méthyle et de chloro-3 propyle sous forme d'une huile orange.

[Rf = 0,3; chromatographie sur couche mince de gel de silice; solvant: cyclohexane-acétate d'éthyle (50-50 en volumes)].

Le dichlorhydrate de (quinolyl-2 méthyl)-2 isothiourée peut être préparé de la manière suivante:

A une suspension de 45,5 g de thiourée dans 260 $cm^3$ d'éthanol bouillant on ajoute goutte à goutte et en 15 minutes une suspension maintenue à 70°C de 107 g de chlorhydrate de chlorométhyl-2 quinoléine dans 450 $cm^3$ d'éthanol. Le mélange réactionnel est agité pendant 1 heure 30 minutes à l'ébullition puis refroidi à 20° C. Les cristaux apparus sont séparés par filtration, lavés 2 fois par 100 $cm^3$ au total d'éthanol et séchés sous pression réduite (20 mm de mercure; 2,7 kPa) à une température voisine de 20° C. On obtient ainsi 141 g de dichlorhydrate de (quinolyl-2 méthyl)-2 isothiourée fondant à 250° C.

## Exemple 2

A une solution de 9 g de (pyridyl-2)-2 tétrahydrothiopyrannecarbodithioate-2 de méthyle dans 60 $cm^3$ d'éthanol maintenue à une température voisine de 20° C, on ajoute goutte à goutte et en 15 minutes 7,5 $cm^3$ d'une solution à 33% (poids/volume) de méthylamine dans l'éthanol. La solution est ensuite agitée pendant 16 heures à une température voisine de 20° C. Les cristaux apparus sont séparés par filtration, lavés 2 fois par 20 $cm^3$ au total d'éthanol et séchés sous pression réduite (20 mm de mercure; 2,7 kPa) à une température voisine de 20° C. On obtient ainsi 7,8 g de produit brut cristallisé que l'on redissout dans 96 $cm^3$ d'éthanol bouillant; la solution ainsi obtenue est additionnée de 0,4 g de noir décolorant, filtrée à chaud puis refroidie pendant 30 minutes à 0° C. Les cristaux apparus sont séparés par filtration, lavés 2 fois par 20 $cm^3$ au total d'éthanol et séchés sous pression réduite (1 mm de mercure; 0,13 kPa) à 45° C. On obtient ainsi 6,3 g de N-méthyl (pyridyl-2)-2 tétrahydrothiopyrannecarbothioamide-2 fondant à 153° C.

Le (pyridyl-2)-2 tétrahydrothiopyrannecarbodithioate-2 de méthyle peut être préparé de la manière suivante:

A 265 $cm^3$ d'une solution 1,6 M de n.butyllithium dans l'hexane maintenue sous atmosphère d'argon

à une température voisine de −60° C, on ajoute goutte à goutte en 10 minutes 192 $cm^3$ d'un mélange d'hexaméthylphosphorotriamide et de tétrahydrofuranne anhydres (47—53 en volumes). On ajoute ensuite en 15 minutes et à la même température une solution de 50,6 g de (pyridyl-2)-2 tétrahydrothiopyranne dans 192 $cm^3$ du mélange d'hexaméthylphosphorotriamide et de tétrahydrofuranne anhydres (47—53 en volumes). Après 5 minutes d'agitation, on ajoute en 13 minutes une solution de 32 g de sulfure de carbone dans 192 $cm^3$ du mélange d'hexaméthylphosphorotriamide et de tétrahydrofuranne anhydres (47—53 en volumes). On ajoute ensuite en 15 minutes une solution de 60,5 g d'iodure de méthyle dans 192 $cm^3$ du mélange d'hexaméthylphosphorotriamide et de tétrahydrofuranne anhydres (47—53 en volumes). Le mélange réactionnel est ensuite agité pendant 45 minutes à −65° C puis pendant 2 heures en laissant remonter progressivement la température à 5° C. Il est ensuite coulé sur un mélange de 1500 $cm^3$ d'eau distillée et de 900 $cm^3$ d'acétate d'éthyle. Après décantation, la phase aqueuse est extraite par 500 $cm^3$ d'acétate d'éthyle. Les extraits organiques sont réunis, lavés 3 fois par 4500 $cm^3$ au total d'eau distillée, séchés sur du sulfate de sodium anhydre, filtrés et concentrés à sec sous pression réduite (20 mm de mercure; 2,7 kPa) à 70° C. Le produit obtenu (87 g) est chromatographié sur 800 g de gel de silice neutre contenus dans une colonne de 6 cm de diamètre; la colonne est éluée par 5700 $cm^3$ d'un mélange cyclohexane-acétate d'éthyle (95-5 en volumes) en recueillant 1 fraction de 1000 $cm^3$, 1 fraction de 800 $cm^3$, 1 fraction de 900 $cm^3$ et 1 fraction de 3000 $cm^3$. Cette dernière est concentrée à sec sous pression réduite (2 mm de mercure; 2,7 kPa) à 50° C. Le produit obtenu (24 g) est dissous dans 100 $cm^3$ d'oxyde d'isopropyle bouillant; la solution est additionnée de 0,4 g de noir décolorant, filtrée à chaud puis refroidie pendant 1 heure à 0° C. Les cristaux apparus sont séparés par filtration, lavés 2 fois par 30 $cm^3$ au total d'oxyde d'isopropyle et séchés sous pression réduite (1 mm de mercure; 0,13 kPa) à 35° C. On obtient ainsi 14,5 g de (pyridyl-2)-2 tétrahydrothiopyrannecarbodithioate-2 de méthyle fondant à 79° C.

Le (pyridyl-2)-2 tétrahydrothiopyranne peut être préparé de la manière suivante:

Une solution de 82 g de sulfure de pyridyl-2 méthyle et de chloro-4 butyle en solution dans 100 $cm^3$ de tétrahydrofuranne anhydre est ajoutée goutte à goutte en 15 minutes et en maintenant la température en-dessous de 30° C, à une solution de 66 g de tertiobutylate de potassium dans un mélange de 100 $cm^3$ d'hexaméthylphosphorotriamide et de 530 $cm^3$ de tétrahydrofuranne anhydres. Après agitation pendant 1 heure à une température voisine de 20° C, on ajoute 20 g de tertiobutylate de potassium et poursuit l'agitation pendant 45 minutes à la même température. Le mélange réactionnel est ajouté à un mélange de 1000 $cm^3$ d'eau distillée et de 600 $cm^3$ d'éther éthylique; après décantation, la phase aqueuse est extraite 2 fois par 400 $cm^3$ au total d'éther éthylique. Les extraits éthérés sont réunis, lavés 3 fois par 3000 $cm^3$ au total d'eau distillée, séchés sur du sulfate de sodium anhydre, filtrés et concentrés sous pression réduite (20 mm de mercure; 2,7 kPa) à 35° C. On obtient ainsi 50,6 g de (pyridyl-2)-2 tétrahydrothiopyranne sous la forme d'une huile brune.

[Rf = 0,7; chromatographie sur couche mince de gel de silice; solvant: cyclohexane-acétate d'éthyle (50-50 en volumes)].

Le sulfure de pyridyl-2 méthyle et de chloro-4 butyle peut être préparé de la manière suivante:

A une solution de 120 g de dichlorhydrate de (pyridyl-2 méthyl)-2 isothiourée dans 250 $cm^3$ d'eau distillée refroidie à 13° C, on ajoute goutte à goutte, en 20 minutes et en maintenant la température en-dessous de 15° C, 100 $cm^3$ d'une solution aqueuse de soude 10 N. Après chauffage pendant 20 minutes à 73° C puis refroidissement à 13° C, on ajoute sous agitation 60 $cm^3$ de solution aqueuse de soude 10 N puis 85 g de bromo-1 chloro-4 butane et poursuit l'agitation pendant 15 heures à une température voisine de 20° C. Le mélange réactionnel est alors extrait 3 fois par 430 $cm^3$ de chlorure de méthylène; les extraits organiques sont réunis, lavés par 300 $cm^3$ d'eau distillée, séchés sur du sulfate de sodium anhydre et filtrés. La solution est versée sur 100 g de gel de silice neutre contenus dans une colonne de 3 cm de diamètre; la colonne est éluée par 3700 $cm^3$ de chlorure de méthylène.

La première fraction (700 $cm^3$) est éliminée. La seconde (2000 $cm^3$) est concentrée à sec sous pression réduite (20 mm de mercure; 2,7 kPa) à 35° C. On obtient ainsi 82 g de sulfure de pyridyl-2 méthyle et de chloro-4 butyle.

[Rf = 0,60; chromatographie sur couche mince de gel de silice; solvant: cyclohexane-acétate d'éthyle (50-50 en volumes)].

Le dichlorhydrate de (pyridyl-2 méthyl)-2 isothiourée peut être préparé de la façon suivante:

A une suspension de 17,6 g de thiourée dans 100 $cm^3$ d'éthanol bouillant on ajoute goutte à goutte et en 15 minutes, 30 g de chlorhydrate de chlorométhyl-2 pyridine en solution dans 100 $cm^3$ d'éthanol à 60° C. L'ébullition est maintenue pendant 90 minutes puis, après refroidissement, les cristaux apparus sont séparés par filtration, lavés 2 fois par 100 $cm^3$ au total d'éthanol et séchés sous pression réduite (20 mm de mercure; 2,7 kPa) à une température voisine de 20° C en présence de potasse en pastilles. On obtient ainsi 41,7 g de dichlorhydrate de (pyridyl-2 méthyl)-2 isothiourée fondant à 220° C.

Le chlorhydrate de chlorométhyl-2 pyridine peut être préparé selon la méthode décrite dans la demande de brevet allemand 1 204 231.

## Exemple 3

A une température voisine de 20° C on sature pendant 45 minutes par un courant de gaz ammoniac anhydre une solution de 10,4 g de (pyridyl-2)-2 dithianne-1,3 carbodithioate-2 de méthyle dans 690 $cm^3$ d'un mélange d'éther éthylique et d'éthanol anhydres (75-25 en volumes). Après 16 heures d'agitation à la même température, le gaz ammoniac est de nouveau admis pendant 1 heure jusqu'à saturation. Les cristaux apparus sont séparés par filtration, lavés 2 fois par 30 $cm^3$ au total d'éther éthylique anhydre et séchés sous pression réduite (20 mm de mercure; 2,7 kPa) à une température voisine de 20° C. On obtient une première fraction de 6,2 g de produit. Le filtrat est concentré sous pression réduite (20 mm de mercure; 2,7 kPa) à 50° C. Après refroidissement on ajoute 100 $cm^3$ d'éther éthylique anhydre au résidu obtenu. Les cristaux apparus sont filtrés, lavés 2 fois par 30 $cm^3$ au total d'éther éthylique anhydre et séchés sous pression réduite (20 mm de mercure; 2,7 kPa) à une température voisine de 20° C. Le produit obtenu (0,5 g) est dissous dans 30 $cm^3$ d'acétonitrile bouillant; la solution est filtrée à chaud puis refroidie à une température voisine de 0° C. Les cristaux apparus sont séparés par filtration, lavés par 3 $cm^3$ d'acétonitrile et séchés sous pression réduite (20 mm de mercure; 2,7 kPa) à une température voisine de 20° C. On obtient ainsi une deuxième fraction de 0,4 g. Les deux fractions (6,2 g et 0,4 g) sont réunies et dissoutes dans 400 $cm^3$ d'acétonitrile bouillant; la solution, additionnée de 0,1 g de noir décolorant est filtrée à chaud puis refroidie pendant 30 minutes à une température voisine de 0° C. Les cristaux apparus sont séparés par filtration, lavés 2 fois par 40 $cm^3$ au total d'acétonitrile et séchés sous pression réduite (1 mm de mercure; 0,13 kPa) à 55° C. On obtient ainsi 5,5 g de (pyridyl-2)-2 dithianne-1,3 carbothioamide-2 fondant à 214° C.

Le (pyridyl-2)-2 dithianne-1,3 carbodithioate-2 de méthyle peut être préparé de la manière suivante:

A 140 $cm^3$ d'une solution 1,6 M de n.butyllithium dans l'hexane, maintenue sous atmosphère d'azote et refroidie à −60° C, on ajoute goutte à goutte et en 10 minutes 140 $cm^3$ d'un mélange d'hexaméthylphosphorotriamide et de tétrahydrofuranne anhydres (47—53 en volumes). On ajoute ensuite en 20 minutes et à la même température une solution de 36 g de (pyridyl-2)-2 dithianne-1,3 dans 140 $cm^3$ du mélange d'hexaméthylphosphorotriamide (47—53 en volumes). Après 15 minutes d'agitation, on ajoute en 10 minutes une solution de 17 g de sulfure de carbone dans 70 $cm^3$ du mélange d'hexaméthylphosphorotriamide et de tétrahydrofuranne anhydres (47—53 en volumes). Après 5 minutes d'agitation à −60° C, on ajoute en 10 minutes une solution de 32 g d'iodure de méthyle dans 70 $cm^3$ d'hexaméthylphosphorotriamide et de tétrahydrofuranne anhydres. Après 1 heure 30 minutes d'agitation à une température voisine de −40° C, on ajoute 500 $cm^3$ d'eau distillée; le mélange est ensuite extrait 4 fois par 1100 $cm^3$ au total d'acétate d'éthyle. Les extraits organiques sont réunis, lavés 3 fois par 1200 $cm^3$ au total d'eau distillée, séchés sur du sulfate de sodium anhydre, filtrés et concentrés à sec sous pression réduite (20 mm de mercure; 2,7 kPa) à 40° C. Le produit obtenu (57 g) est chromatographié sur 1080 g d'alumine neutre contenus dans une colonne de 5,2 cm de diamètre. On élue avec 6000 $cm^3$ de cyclohexane en recueillant des fractions de 1000 $cm^3$ puis avec 2500 $cm^3$ d'un mélange cyclohexaneacétate d'éthyle (99-1 en volumes) en recueillant des fractions de 500 $cm^3$. Les fractions 7 à 11 sont réunies et concentrées à sec sous pression réduite (20 mm de mercure; 2,7 kPa) à 40° C. Le produit obtenu (15 g) est dissous dans 30 $cm^3$ d'éthanol bouillant et la solution est refroidie pendant 1 heure à 0° C. Les cristaux apparus sont séparés par filtration, lavés 2 fois par 20 $cm^3$ au total d'éthanol et séchés sous pression réduite (20 mm de mercure; 2,7 kPa) à une température voisine de 20° C. On obtient ainsi 7,7 g de (pyridyl-2)-2 dithianne-1,3 carbodithioate-2 de méthyle fondant à 91° C.

Le (pyridyl-2)-2 dithianne-1,3 peut être préparé de la façon suivante:

Une solution de 26,7 g de pyridinecarboxaldéhyde-2, de 94,4 g de propanedithiol-1,3 et de 3,7 g d'acide paratoluènesulfonique dans 2500 $cm^3$ de dichloro-1,2 éthane est maintenue à l'ébullition pendant 20 heures de façon à éliminer par azéotropie l'eau formée. Après refroidissement à 5° C, le mélange réactionnel est lavé 2 fois par 540 $cm^3$ au total d'une solution aqueuse environ 7 N de potasse puis 4 fois par 1400 $cm^3$ au total d'eau distillée. La solution organique est séchée sur du sulfate de sodium anhydre, filtrée et concentrée sous pression réduite (20 mm de mercure; 2,7 kPa) à 40° C. On obtient ainsi 41,9 g de (pyridyl-2)-2 dithianne-1,3.

[Rf=0,6; chromatographie sur couche mince de gel de silice; solvant: cyclohexane-acétate d'éthyle (50-50 en volumes)].

## Exemple 4

A une solution de 17,5 g de (pyridyl-2)-2 oxathianne-1,3 carbodithioate-2 de méthyle dans 60 $cm^3$ d'éthanol, on ajoute goutte à goutte, en 10 minutes et à une température comprise entre 34° C et 37° C, 6,5 $cm^3$ d'une solution à 33% (poids/volume) de méthylamine dans l'éthanol. Le mélange réactionnel est ensuite agité pendant 30 minutes à 2° C. Les cristaux apparus sont séparés par filtration, lavés 2 fois par 40 $cm^3$ au total d'éther de pétrole et séchés sous pression réduite (20 mm de mercure; 2,7 kPa) à

une température voisine de 20°C. Le produit obtenu (13,4 g) est dissous dans 80 $cm^3$ d'éthanol bouillant; la solution est additionnée de 0,2 g de noir décolorant, filtrée à chaud puis refroidie pendant 1 heure à une température voisine de 0°C. Les cristaux apparus sont séparés par filtration, lavés 2 fois par 20 $cm^3$ au total d'éthanol et séchés sous pression réduite (1 mm de mercure; 0,13 kPa) à 50°C. On obtient ainsi 12 g de N-méthyl (pyridyl-2)-2 oxathianne-1,3 carbothioamide-2 fondant à 157°C.

Le (pyridyl-2)-2 oxathianne-1,3 carbodithioate-2 de méthyle peut être préparé de la manière suivante:

A 212 $cm^3$ d'une solution 1,6 M de n.butyllithium dans l'hexane, maintenue sous atmosphère d'azote et refroidie à une température voisine de −60°C, on ajoute goutte à goutte et en 15 minutes 153 $cm^3$ d'un mélange d'hexaméthylphosphorotriamide et de tétrahydrofuranne anhydres (47—53 en volumes). On ajoute ensuite en 25 minutes à la même température une solution de 34,4 g de (pyridyl-2)-2 oxathianne-1,3 dans 153 $cm^3$ du mélange d'hexaméthylphosphorotriamide et de tétrahydrofuranne anhydres (47—53 en volumes). Après 15 minutes d'agitation,on ajoute, en 15 minutes, une solution de 23,3 g de sulfure de carbone dans 153 $cm^3$ du mélange d'hexaméthylphosphorotriamide et de tétrahydrofuranne anhydres (47—53 en volumes). Après 5 minutes d'agitation à −70°C, on ajoute en 10 minutes une solution de 43,4 g d'iodure de méthyle dans 153 $cm^3$ d'hexaméthylphosphorotriamide et de tétrahydrofuranne anhydres (47—53 en volumes). Le mélange réactionnel est agité pendant 45 minutes à −70°C puis pendant 1 heure en laissant remonter progressivement la température à 20°C environ. Le mélange réactionnel est coulé sur un mélange de 1200 $cm^3$ d'eau distillée et de 800 $cm^3$ d'acétate d'éthyle. Après décantation, la phase aqueuse est extraite 2 fois par 1200 $cm^3$ au total d'acétate d'éthyle; les phases organiques sont réunies, lavées 3 fois par 1500 $cm^3$ au total d'eau distillée, séchées sur du sulfate de sodium anhydre, filtrées et concentrées à sec sous pression réduite (20 mm de mercure; 2,7 kPa) à 45°C. Le produit obtenu (69,3 g) est chromatographié sur 700 g de gel de silice neutre contenus dans une colonne de 5,4 cm de diamètre. On élue successivement avec 2300 $cm^3$ de cyclohexane, 4000 $cm^3$ d'un mélange cyclohexane-acétate d'éthyle (98-2 en volumes), 7000 $cm^3$ d'un mélange cyclohexane-acétate d'éthyle (95-5 en volumes) et 3000 $cm^3$ d'un mélange cyclohexaneacétate d'éthyle (90-10 en volumes) en recueillant une première fraction de 2300 $cm^3$ puis 14 fractions de 1000 $cm^3$. Les fractions 11 à 15 sont réunies et concentrées à sec sous pression réduite (20 mm de mercure; 2,7 kPa) à 45°C. On obtient ainsi 17,5 g de (pyridyl-2)-2 oxathianne-1,3 carbodithioate-2 de méthyle brut fondant à 116°C.

Le (pyridyl-2)-2 oxathianne-1,3 peut être préparé de la façon suivante:

Une solution de 26,7 g de pyridinecarboxaldéhyde-2, de 81,1 g de mercapto-3 propanol-1 et de 3,7 g d'acide paratoluènesulfonique dans 2500 $cm^3$ de dichloro-1,2 éthane est maintenue à l'ébullition pendant 15 heures de façon à éliminer par azéotropie l'eau formée. Après refroidissement à une température voisine de 20°C, le mélange réactionnel est lavé 3 fois par 1200 $cm^3$ au total d'une solution aqueuse 5 N de soude puis 3 fois par 2400 $cm^3$ au total d'eau distillée. La phase organique est séchée sur du sulfate de sodium anhydre, filtrée et concentrée sous pression réduite (20 mm de mercure; 2,7 kPa) à 50°C. On obtient 36,6 g de (pyridyl-2)-2 oxathianne-1,3 sous la forme d'une huile brunclair.

[Rf=0,45; chromatographie sur couche mince de gel de silice; solvant: cyclohexane-acétate d'éthyle (50-50 en volumes)].

Le mercapto-3 propanol-1 peut être préparé comme décrit dans la littérature [R. O. CLINTON et coll. J. Am. Chem. Soc., 67, 594 (1945)].

Exemple 5

A 300 $cm^3$ d'une solution 1,6 M de n.butyllithium dans l'hexane maintenue sous atmosphère d'argon à une température voisine de −60°C, on ajoute goutte à goutte et en 22 minutes, 225 $cm^3$ d'un mélange d'hexaméthylphosphorotriamide et de tétrahydrofuranne anhydres (47—53 en volumes). On ajoute ensuite en 30 minutes une solution de 56 g de (pyridyl-2)-2 dithianne-1,3 dans 225 $cm^3$ du mélange d'hexam éthylphosphorotriamide et de tétrahydrofuranne anhydres (47—53 en volumes). Après 15 minutes d'agitation, on ajoute en 15 minutes 31 g d'isothiocyanate de méthyle en solution dans 225 $cm^3$ du mélange d'hexaméthylphosphorotriamide et de tétrahydrofuranne anhydres (47—53 en volumes). Le mélange réactionnel est ensuite agité pendant 1 heure à −65°C puis pendant 1 heure en laissant remonter progressivement la température à 20°C environ. Il est ensuite coulé sur un mélange de 1500 $cm^3$ d'eau distillée et de 1500 $cm^3$ d'acétate d'éthyle. Après décantation, la solution aqueuse est extraite 2 fois par 2500 $cm^3$ au total d'acétate d'éthyle. Les extraits organiques sont réunis, lavés 3 fois par 4500 $cm^3$ au total d'eau distillée, séchés sur du sulfate de sodium anhydre, filtrés et concentrés à sec sous pression réduite (20 mm de mercure; 2,7 kPa) à 50°C. Le produit obtenu (90 g) est dissous dans 600 $cm^3$ d'éthanol bouillant et la solution est refroidie pendant 30 minutes à une température voisine de 5°C. Les cristaux apparus sont séparés par filtration, lavés par 20 $cm^3$ d'éthanol puis 2 fois par 40 $cm^3$ au total d'oxyde d'isopropyle et séchés sous pression réduite (20 mm de mercure; 2,7 kPa) à une température voisine de 20°C. Le produit obtenu (23,7 g) réuni à 0,7 g d'un produit préparé dans les

mêmes conditions dans une autre opération similaire, est dissous dans 780 $cm^3$ d'éthanol bouillant; la solution est additionnée de 2,5 g de noir décolorant, filtrée à chaud puis refroidie pendant 1 heure à une température voisine de 5° C. Les cristaux apparus sont séparés par filtration, lavés 2 fois par 50 $cm^3$ au total d'éthanol puis par 25 $cm^3$ d'oxyde d'isopropyle et séchés sous pression réduite (20 mm de mercure; 2,7 kPa) à une température voisine de 20° C. Le produit ainsi obtenu (19 g) est dissous dans 600 $cm^3$ d'éthanol bouillant; la solution est additionnée de 5 g de noir décolorant, filtrée à chaud puis refroidie pendant 15 heures à une température voisine de 5° C. Les cristaux apparus sont séparés par filtration, lavés 2 fois par 50 $cm^3$ au total d'éthanol puis par 25 $cm^3$ d'oxyde d'isopropyle et séchés sous pression réduite (1 mm de mercure; 0,13 kPa) à 55° C. On obtient ainsi 15,9 g de N-méthyl (pyridyl-2)-2 dithianne-1,3 carbothioamide-2 fondant à 159° C.

Exemple 6

A 75 $cm^3$ d'une solution 1,6 M de n.butyllithium dans l'hexane, maintenue sous atmosphère d'azote à une température voisine de −60° C, on ajoute goutte à goutte et en 15 minutes 80 $cm^3$ d'un mélange d'hexaméthylphosphorotriamide et de tétrahydrofuranne anhydres (47—53 en volumes). On ajoute ensuite en 20 minutes une solution de 15 g de (pyridyl-2)-2 tétrahydrofuranne dans 80 $cm^3$ du mélange d'hexaméthylphosphorotriamide et de tétrahydrofuranne anhydres (47—53 en volumes). Après 30 minutes d'agitation à la même température, on ajoute en 20 minutes une solution de 8,8 g d'isothiocyanate de méthyle dans 80 $cm^3$ d'un mélange d'hexaméthylphosphorotriamide et de tétrahydrofuranne anhydres (47—53 en volumes). Le mélange réactionnel est ensuite agité pendant 45 minutes à environ −60° C puis pendant 1 heure en laissant remonter progressivement la température à 20° C environ. Il est ensuite coulé sur un mélange de 600 $cm^3$ d'eau distillée et de 400 $cm^3$ d'acétate d'éthyle. Après décantation, la solution aqueuse est extraite 2 fois par 800 $cm^3$ au total d'acétate d'éthyle. Les extraits organiques sont réunis, lavés 3 fois par 1500 $cm^3$ au total d'eau distillée, séchés sur du sulfate de sodium anhydre, filtrés et concentrés sous pression réduite (20 mm de mercure; 2,7 kPa) à 45° C. Le produit obtenu (6,6 g) est dissous dans 350 $cm^3$ d'oxyde d'isopropyle bouillant; la solution est additionnée de 0,5 g de noir décolorant, filtrée à chaud puis refroidie pendant 30 minutes à 0° C. Les cristaux apparus sont séparés par filtration, lavés 2 fois par 10 $cm^3$ au total d'oxyde d'isopropyle et séchés sous pression réduite (20 mm de mercure; 2,7 kPa) à une température voisine de 20° C. Le produit obtenu (7,7 g) réuni à 0,8 g de produit préparé dans les mêmes conditions est dissous dans 30 $cm^3$ d'éthanol bouillant; la solution est additionnée de 0,2 g de noir décolorant, filtrée à chaud puis refroidie pendant 1 heure à 0° C. Les cristaux apparus sont séparés par filtration, lavés 2 fois par 10 $cm^3$ au total d'éthanol et séchés sous pression réduite (1 mm de mercure; 0,13 kPa) à 45° C. On obtient ainsi 6 g de N-méthyl (pyridyl-2)-2 tétrahydrofurannecarbothioamide-2 fondant à 115° C.

Le (pyridyl-2)-2 tétrahydrofuranne peut être préparé de la manière suivante:

Une solution de 122 g de (diméthyl-1,1 propoxy)-4 (pyridyl-2)-1 butanol-1 et de 107,4 g d'acide paratoluènesulfonique dans 1000 $cm^3$ de toluène est maintenue à l'ébullition pendant 28 heures de façon à éliminer par azéotropie l'eau formée. Après refroidissement à une température voisine de 20° C, on ajoute 250 $cm^3$ d'eau distillée. La phase organique est décantée et lavée par 50 $cm^3$ d'eau distillée; les phases aqueuses sont réunies et neutralisées par addition de 49 g de bicarbonate de sodium. Le mélange est extrait 3 fois par 1500 $cm^3$ au total d'acétate d'éthyle; les extraits organiques sont réunis, filtrés et concentrés sous pression réduite (20 mm de mercure; 2,7 kPa) à 4C° C. On obtient ainsi une première fraction huileuse de 30,8 g. La phase aqueuse précédente est à nouveau extraite 5 fois par 2500 $cm^3$ au total de chlorure de méthylène; les extraits organiques sont réunis, séchés sur du sulfate de sodium anhydre, filtrés et concentrés à sec sous pression réduite (20 mm de mercure; 2,7 kPa) à 35° C. On obtient ainsi une deuxième fraction huileuse de 11,2 g. Les 2 fractions (30,8 g et 11,2 g) sont réunies, coulées en 30 minutes sur 200 g d'acide polyphosphorique à 80° C. Après 10 minutes d'agitation à la même température puis refroidissement à une température voisine de 20° C, le mélange réactionnel est versé dans 400 $cm^3$ d'eau distillée. On ajoute ensuite 450 $cm^3$ d'une solution aqueuse de soude 10 N en maintenant la température inférieure à 20° C. Les cristaux apparus sont séparés par filtration et lavés par 500 $cm^3$ d'acétate d'éthyle. Après décantation du filtrat, la phase aqueuse est extraite 3 fois par 1500 $cm^3$ au total d'acétate d'éthyle. Les extraits organiques sont réunis, séchés sur sulfate de sodium anhydre, filtrés et concentrés sous pression réduite (20 mm de mercure; 2,7 kPa) à 40° C. On obtient ainsi 26 g de (pyridyl-2)-2 tétrahydrofuranne.

[Rf = 0,57; chromatographie sur couche mince de gel de silice; solvant: acétate d'éthyle]

Le (diméthyl-1,1 propoxy)-4 (pyridyl-2)-1 butanol-1 peut être préparé selon la méthode décrite dans la littérature pour le (diméthyl-1,1 propoxy)-4 phényl-1 butanol-1 [W. B. RENFROW et coll., J. Org. Chem. 26, 935 (1961)].

La présente invention concerne egalement les médicaments constitués par au moins un produit de formule générale (I), à l'état pur ou sous forme d'une composition dans laquelle il est associé à tout

autre produit pharmaceutiquement compatible, pouvant être inerte ou physiologiquement actif. Les médicaments selon l'invention peuvent être utilisés par voie orale, parentérale ou rectale.

Comme compositions solides pour administration orale peuvent être utilisés des comprimés, pilules, poudres (notamment dans des capsules de gélatine ou des cachets) ou granulés. Dans ces compositions, le produit actif selon l'invention est mélangé à un ou plusieurs diluants inertes, tels que amidon, cellulose, saccharose, lactose ou silice. Ces compositions peuvent également comprendre des substances autres que les diluants, par exemple un ou plusieurs lubrifiants tel que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent également comprendre des substances autres que les diluants, par exemple des produits mouillants, édulcorants, épaississants, aromatisants ou stabilisants.

Les compositions stériles pour administration parentérale peuvent être de préférence des solutions aqueuses ou non aqueuses, des suspensions ou des émulsions. Comme solvant ou véhicle, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont les suppositoires ou les capsules rectales, qui contiennent outre le produit actif des excipients tels que le beurre de cacao, des glycérides semi-synthétiques ou des polyéthylèneglycols.

En thérapeutique humaine, les produits de l'invention sont, selon la définition du symbole Het donnée précédemment, particulièrement utiles dans le traitement des ulcères gastrointestinaux. Les doses dépendent de l'effet recherché et de la durée du traitement; elles sont généralement comprises entre 25 et 1000 mg par jour par voie orale pour un adulte en une ou plusieurs prises.

D'une façon générale, le médecin déterminera la posologie qu'il estime la plus appropriée en fonction de l'âge, du poids et de tous les autres facteurs propres au sujet à traiter.

Les exemples suivants, donnés à titre non limitatif, illustrent des compositions selon l'invention.

Exemple A —

On prépare, selon la technique habituelle, des comprimés dosés à 50 mg de produit actif ayant la composition suivante:

| | |
|---|---|
| — N-méthyl (pyridyl-2)-2 dithianne-1,3 carbothioamide-2 | 50 mg |
| — amidon | 60 mg |
| — lactose | 50 mg |
| — stéarate de magnésium | 2 mg |

**Revendications pour les Etats contractants: BE, CH, DE, FR, GB, IT, LI, LU, NL, SE**

1. Un nouveau dérivé de la thioformamide caractérisé en ce qu'il répond à la formule générale:

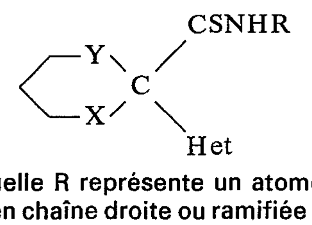

dans laquelle R représente un atome d'hydrogène ou un radical alcoyle contenant 1 à 4 atomes de carbone en chaîne droite ou ramifiée et

— soit Het représente le radical quinolyle-2, X représente un atome de soufre et Y représente une liaison de valence,
— soit Het représente le radical pyridyle-2, X représente un atome de soufre ou d'oxygène et Y représente un atome de soufre ou d'oxygène ou un radical méthylène,
— soit Het représente le radical pyridyle-2, X représente un atome d'oxygène et Y représente une liaison de valence.

2. Procédé de préparation d'un produit selon la revendication 1 caractérisé en ce que l'on fait réagir une amine de formule générale:

$R-NH_2$

dans laquelle R est défini comme dans la revendication 1 sur un dithioester de formule générale:

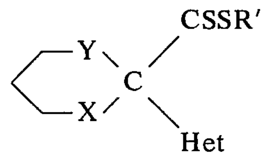

dans laquelle les symboles Het, X et Y sont définis comme dans la revendication 1 et R' représente un radical alcoyle contenant 1 à 4 atomes de carbone en chaîne droite ou ramifiée ou un radical benzyle ou carboxyméthyle,
puis isole le produit obtenu.

3. Procédé de préparation d'un produit selon la revendication 1 dans la formule duquel R est un radical alcoyle contenant 1 à 4 atomes de carbone en chaîne droite ou ramifiée caractérisé en ce que, sur un dérivé de formule générale:

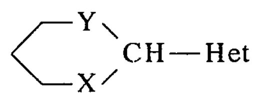

dans laquelle X, Y et Het sont définies comme dans la revendication 1, on fait réagir un dérivé organo-lithien puis un isothiocyanate de formule générale:

$R'''-N=C=S$

dans laquelle R''' représente un radical alcoyle contenant 1 à 4 atomes de carbone en chaîne droite ou ramifiée, puis isole le produit obtenu.

4. Médicament caractérisé en ce qu'il consiste en un produit selon la revendication 1.

5. Composition pharmaceutique caractérisée en ce qu'elle contient à titre de produit actif au moins un produit selon la revendication 1 éventuellement en association avec un ou plusieurs diluants ou adjuvants compatibles et pharmaceutiquement acceptables.

**Revendication pour l'Etat contractant: AT**

1. Procédé de préparation d'un nouveau dérivé de la thioformamide de formule générale:

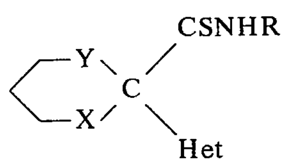

dans laouelle R représente un atome d'hydrogène ou un radical alcoyle contenant 1 à 4 atomes de carbone en chaîne droite ou ramifiée et

- soit Het représente le radical quinolyle-2, X représente un atome de soufre et Y représente une liaison de valence,
- soit Het représente le radical pyridyle-2, X représente un atome de soufre ou d'oxygène et Y représente un atome de soufre ou d'oxygène ou un radical méthylène,
- soit Het représente le radical pyridyle-2, X représente un atome d'oxygène et Y représente une liaison de valence,

caractérisé en ce que l'on fait réagir une amine de formule générale:

$R-NH_2$

dans laquelle R a la définition correspondante sur un dithioester de formule générale:

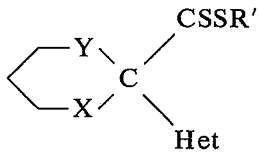

dans laquelle les symboles Het, X et Y sont définis comme précédemment et R′ représente un radical alcoyle contenant 1 à 4 atomes de carbone en chaîne droite ou ramifiée ou un radical benzyle ou carboxyméthyle,
puis isole le produit obtenu,
ou, lorsque R est un radical alcoyle contenant 1 à 4 atomes de carbone en chaîne droite ou ramifiée en ce que, sur un dérivé de formule générale:

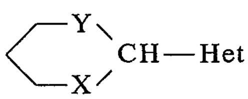

dans laquelle X, Y et Het sont définis comme précédemment, on fait réagir un dérivé organo-lithien puis un isothiocyanate de formule générale:

$$R'''-N=C=S$$

dans laquelle R‴ représente un radical alcoyle contenant 1 à 4 atomes de carbone en chaîne droite ou ramifiée, puis isole le produit obtenu.

**Patentansprüche für die Vertragsstaaten: BE, CH, DE, FR, GB, IT, LI, LU, NL, SE**

1. Ein neues Derivat des Thioformamids, dadurch gekennzeichnet, daß es der allgemeinen Formel

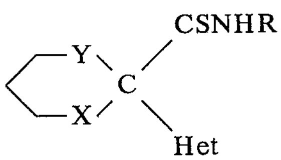

entspricht, worin R ein Wasserstoffatom oder einen Alkylrest mit 1 bis 4 Kohlenstoffatomen in gerader oder verzweigter Kette bedeutet und

- entweder Het den Chinolyl-2-Rest bedeutet, X ein Schwefelatom ist und Y eine Valenzbindung darstellt,
- oder Het den Pyridyl-2-Rest, X ein Schwefel- oder Sauerstoffatom und Y ein Schwefel- oder Sauerstoffatom oder einen Methylenrest bedeutet,
- oder Het den Pyridyl-2-Rest, X ein Sauerstoffatom und Y eine Valenzbindung bedeutet.

2. Verfahren zur Herstellung eines Produktes gemäß Anspruch 1, dadurch gekennzeichnet, daß man ein Amin der allgemeinen Formel

$$R-NH_2$$

worin R wie in Anspruch 1 definiert ist, auf einen Dithioester der allgemeinen Formel

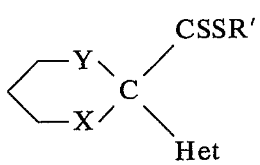

einwirken läßt, worin die Symbole Het, X und Y wie in Anspruch 1 definiert sind und R′ einen Alkylrest mit 1 bis 4 Kohlenstoffatomen in gerader oder verzweigter Kette oder einen Benzyl- oder Carboxymethylrest bedeutet,
und daß man dann das erhaltene Produkt isoliert.

3. Verfahren zur Herstellung eines Produktes gemäß Anspruch 1, in dessen Formel R einen Alkylrest mit 1 bis 4 Kohlenstoffatomen in gerader oder verzweigter Kette darstellt, dadurch gekennzeichnet, daß man auf ein Derivat der allgemeinen Formel

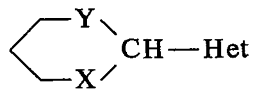

worin X, Y und Het wie in Anspruch 1 definiert sind, ein Organolithium-Derivat und dann ein Isothiocyanat der allgemeinen Formel

$R''' - N = C = S$

einwirken läßt, worin R''' einen Alkylrest mit 1 bis 4 Kohlenstoffatomen in gerader oder verzweigter Kette darstellt, und daß man das erhaltene Produkt dann isoliert.

4. Arzneimittel, dadurch gekennzeichnet, daß es aus einem Produkt gemäß Anspruch 1 besteht.

5. Pharmazeutische Zusammensetzung, dadurch gekennzeichnet, daß sie als aktives Produkt mindestens ein Produkt gemäß Anspruch 1, gegebenenfalls in Verbindung mit einem oder mehreren verträglichen und pharmazeutisch annehmbaren Verdünnungsmitteln oder Hilfsmitteln, enthält.

**Patentanspruch für den Vertragsstaat: AT**

Verfahren zur Herstellung eines neuen Derivats des Thioformamids der allgemeinen Formel

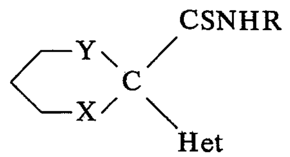

worin R ein Wasserstoffatom oder einen Alkylrest mit 1 bis 4 Kohlenstoffatomen in gerader oder verzweigter Kette bedeutet und

- entweder Het den Chinolyl-2-Rest, bedeutet, X ein Schwefelatom darstellt und Y eine Valenzbindung ist,
- oder Het den Pyridyl-2-Rest, X ein Schwefel- oder Sauerstoffatom und Y ein Schwefel- oder Sauerstoffatom oder einen Methylenrest bedeutet,
- oder Het den Pyridyl-2-Rest bedeutet, X ein Sauerstoffatom darstellt und Y eine Valenzbindung bedeutet,

dadurch gekennzeichnet, daß man ein Amin der allgemeinen Formel

$R - NH_2$

worin R die entsprechende Definition hat, auf einen Dithioester der allgemeinen Formel

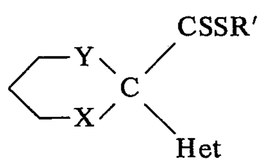

einwirken läßt, worin die Symbole Het, X und Y wie vorstehend definiert sind und R' einen Alkylrest mit 1 bis 4 Kohlenstoffatomen in gerader oder verzweigter Kette oder einen Benzyl- oder Carboxymethylrest bedeutet,
und daß man das erhaltene Produkt dann isoliert,
oder daß man, falls R ein Alkylrest mit 1 bis 4 Kohlenstoffatomen in gerader oder verzweigter Kette ist, auf ein Derivat der allgemeinen Formel

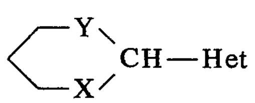

worin X, Y und Het wie vorstehend definiert sind, ein Organolithiumderivat und dann ein Isothiocyanat der allgemeinen Formel

$R''' - N = C = S$

einwirken läßt, worin R‴ einen Alkylrest mit 1 bis 4 Kohlenstoffatomen in gerader oder verzweigter Kette darstellt, und daß man das erhaltene Produkt dann isoliert.

**Claims for the contracting States: BE, CH, DE, FR, GB, IT, LI, LU, NL, SE**

1. A new thioformamide derivative, characterised in that it corresponds to the general formula:

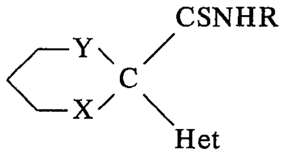

in which R represents a hydrogen atom or an alkyl radical containing 1 to 4 carbon atoms in a linear or branched chain and

— Het represents the quinolin-2-yl radical, X represents a sulphur atom and Y represents a valence bond,
— or Het represents the pyridin-2-yl radical, X represents a sulphur or oxygen atom and Y represents a sulphur or oxygen atom or a methylene radical,
— or Het represents the pyridin-2-yl radical, X represents an oxygen atom and Y represents a valence bond.

2. Process for the preparation of a product according to Claim 1, characterised in that an amine of the general formula:

$R-NH_2$

in which R is defined as in Claim 1, is reacted with a dithioester of the general formula:

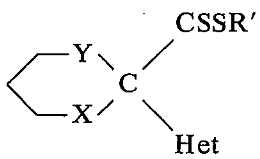

in which the symbols Het, X and Y are defined as in Claim 1 and R′ represents an alkyl radical containing 1 to 4 carbon atoms in a linear or branched chain, or a benzyl or carboxymethyl radical, and the product obtained is then isolated.

3. Process for the preparation of a product according to Claim 1 in the formula of which R ist an alkyl radical containing 1 to 4 carbon atoms in a linear or branched chain, characterised in that an organolithium derivative and then an isothiocyanate of the general formula:

$R'''-N=C=S,$

in which R‴ represents an alkyl radical containing 1 to 4 carbon atoms in a linear or branched chain, are reacted with a derivative of the general formula:

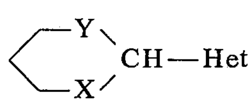

in which X, Y and Het are defined as in Claim 1, and the product obtained is then isolated.

4. Medicament, characterised in that it consists of a product according to Claim 1.

5. Pharmaceutical composition, characterised in that it contains, as the active product, at least one product according to Claim 1, if appropriate in association with one or more compatible and pharmaceutically acceptable diluents or adjuvants.

## Claim for the contracting State: AT

1. Process for the preparation of a new thioformamide derivative of the general formula:

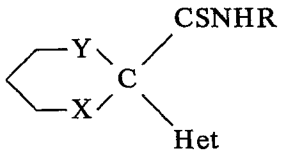

in which R represents a hydrogen atom or an alkyl radical containing 1 to 4 carbon atoms in a linear or branched chain and

- Het represents the quinolin-2-yl radical, X represents a sulphur atom and Y represents a valence bond,
- or Het represents the pyridin-2-yl radical, X represents a sulphur or oxygen atom and Y represents a sulphur or oxygen atom or a methylene radical,
- or Het represents the pyridin-2-yl radical, X represents an oxygen atom and Y represents a valence bond,

characterised in that an amine of the general formula:

$R-NH_2$,

in which R has the corresponding definition, is reacted with a dithioester of the general formula:

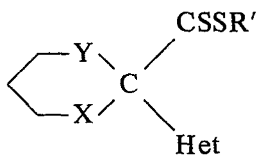

in which the symbols Het, X and Y are defined as above and R′ represents an alkyl radical containing 1 to 4 carbon atoms in linear or branched chain, or a benzyl or carboxymethyl radical, and the product obtained is then isolated, or, if R is an alkyl radical containing 1 to 4 carbon atoms in a linear or branched chain, in that an organolithium derivative and then an isothiocyanate of the general formula:

$R'''-N=C=S$,

in which R‴ represents an alkyl radical containing 1 to 4 carbon atoms in a linear or branched chain, are reacted with a derivative of the general formula:

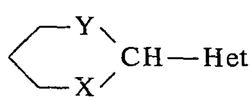

in which X, Y and Het are defined as above, and the product obtained is then isolated.